Europäisches Patentamt

(19) European Patent Office

Office européen des brevets

(11) Veröffentlichungsnummer: **0 010 584**
**B1**

# (12) EUROPÄISCHE PATENTSCHRIFT

(45) Veröffentlichungstag der Patentschrift:
23.06.82

(21) Anmeldenummer: 79103109.9

(22) Anmeldetag: 23.08.79

(51) Int. Cl.³: **G 01 L 27/00,** G 05 D 16/20,
A 61 M 1/03

(54) **Vorrichtung zur Drucküberwachung.**

(30) Priorität: 19.10.78 US 952766

(43) Veröffentlichungstag der Anmeldung:
14.05.80 Patentblatt 80/10

(45) Bekanntmachung des Hinweises auf die Patenterteilung:
23.06.82 Patentblatt 82/25

(84) Benannte Vertragsstaaten:
AT CH DE FR GB SE

(56) Entgegenhaltungen:
DE-A1-2 738 163
DE-A1-2 738 164
DE-A1-2 844 105
US-A-3 946 731
US-A-4 080 966

(73) Patentinhaber: **International Business Machines Corporation, Armonk, N.Y. 10504 (US)**

(72) Erfinder: **Mulzet, Alfred Paul, 16 Dover Drive, Endicott, NY 13760 (US)**
Erfinder: **Trudgen, Gary Allen, 3103 Chatham Road, Endwell, NY 13760 (US)**

(74) Vertreter: **Böhmer, Hans Erich, Dipl.-Ing., Schönaicher Strasse 220, D-7030 Böblingen (DE)**

Vorrichtung zur Drucküberwachung

Die Erfindung betrifft eine Vorrichtung für die Überwachung des Druckes eines strömenden flüssigen Mediums in einem biegsamen Rohr oder Schlauch zur Feststellung einer Abweichung des jeweiligen Istwertes des Druckes von durch einen oberen und einen unteren Bezugssignalwert begrenzten Sollwerten, mit einem an das Rohr oder den Schlauch angekoppelten druckempfindlichen Wandler, Vergleichsschaltungen zum Vergleich des Ausgangssignals des Wandlers mit dem oberen und unteren Bezugssignalwert, sowie einer Steuerschaltung zur Einstellung der Bezugssignalwerte in Abhängigkeit vom Istwert des Druckes während einer ersten Betriebsphase und zur Bereithaltung der eingestellten Bezugssignalwerte während einer zweiten Betriebsphase.

Beschreibung des Standes der Technik

Druckwandler unter Verwendung von Dehnungsmeßstreifen, die an Brückenschaltungen angeschlossen sind, sind beispielsweise in den US-Patentschriften 3 535 937 und 3 866 473 offenbart. Die US-Patentschrift 3 946 731 schlägt die Überwachung und Regelung eines außerhalb der Körpers verlaufenden Kreislaufs durch mindestens ein Druckabfühlelement vor.

Aus der DE-A 2 738 164 ist außerdem eine Blutdruck-Alarmvorrichtung für ein Dialysegerät bekannt, das mit einem Druckwandler und einem damit verbundenen Speicher- und Überwachungssystem arbeitet. Zu Beginn wird dabei die an einem Druckwandler abgenommene Spannung nach Verstärkung einmal unmittelbar einem anzeigenden Meßgerät und außerdem einer Vergleichsstufe zur Steuerung der Zählung der von einem Impulsgenerator kommenden Impulse in einem Aufwärts/Abwärtszähler über eine Steuerstufe zugeführt. Sobald der Zähler den der Ausgangsspannung des Druckwandlers entsprechenden digitalen Zählerstand erreicht hat, kann durch Betätigen eines Schalters die weitere Zählung gesperrt werden. Ohne diese Sperrung würde der Zähler bei gleichbleibendem Druck durch ständiges Umschalten zwischen Aufwärts- und Abwärtszählen pendeln.

Der nach Sperrung festgehaltene Zählwert dient dann als Sollwert, dessen Toleranzgrenzen durch eine einstellbare Druckänderungswahlschaltung festgelegt werden können.

Mit dieser Vorrichtung ist jedoch nicht feststellbar, ob der Schlauch mit dem darin befindlichen flüssigen Medium, dessen Druck gemessen und überwacht werden soll, auch richtig eingelegt ist.

Die der Erfindung zugrundeliegende Aufgabe besteht nun darin, eine Vorrichtung der eingangs genannten Art so zu verbessern, daß bereits in einer Anlaufphase festgestellt werden kann, ob der Schlauch richtig in die den Wandler tragende Vorrichtung eingelegt worden ist.

Dies wird erfindungsgemäß dadurch erreicht, daß ein Stützblock mit einem für die Aufnahme des Rohrs oder Schlauchs bestimmten Kanal vorgesehen ist, in dessen Seitenwand der Wandler in der Weise angeordnet ist, daß das in den Stützblock eingelegte Rohr bzw. der Schlauch einen vorbestimmten Vorlastdruck auf den Wandler ausübt, daß die Steuerschaltung eine weitere Vergleichsschaltung aufweist, die ein in Abhängigkeit von ihrem Ausgangssignal erzeugtes, der Einstellung der Bezugssignalwerte dienendes Steuersignal mit dem Ausgangssignal des Wandlers vergleicht, daß eine logische Schaltung zur Feststellung und Anzeige der Unter- bzw. Überschreitung eines vorgegebenen unteren bzw. oberen Grenzwertes durch das Steuersignal während der ersten Betriebsphase vorgesehen ist, wobei der obere und untere Grenzwert in Abhängigkeit von den Bereichsgrenzen des zulässigen Vorlastdrucks gewählt sind.

Weitere Ausgestaltungen der Erfindung sind in den Unteransprüchen gekennzeichnet.

Die Erfindung wird nunmehr anhand eines bevorzugten Ausführungsbeispiels der Erfindung in Verbindung mit den Zeichnungen näher erläutert.

In den Zeichnungen zeigt

Fig. 1 ein stark schematisiertes Blockschaltbild eines außerhalb des Körpers befindlichen Kreislaufsystems, bei dem eine Drucküberwachung gemäß der Erfindung eingesetzt werden kann,

Fig. 2 schematisch die wesentlichen Beziehungen zwischen dem Schlauch, dem Druckmeßblock, dem Wandler und dem Verstärker, und

Fig. 3 ein Blockschaltbild des Verstärkers der Drucküberwachungsvorrichtung.

Für gleiche Teile in den verschiedenen Ansichten sind dabei gleiche Bezugszeichen verwendet worden.

Einzelbeschreibung einer bevorzugten
Ausführungsform

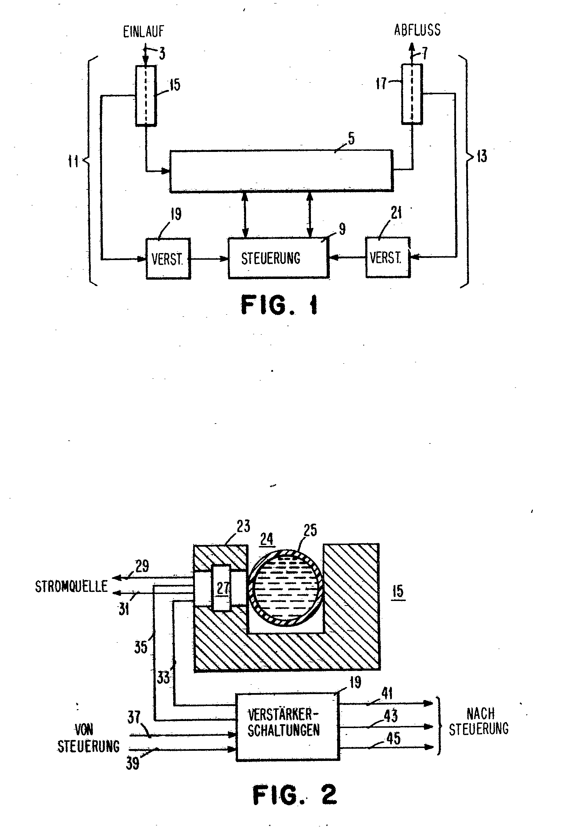

Fig. 1 zeigt ein stark vereinfachtes Blockschaltbild eines außerhalb des Körpers befindlichen Kreislaufsystems, bei dem die Erfindung verwendet werden kann. Die Bahn der Flüssigkeit oder der Kreislauf verläuft von einer Einlaßleitung 3 durch verschiedene zur Behandlung der Flüssigkeit vorgesehene Vorrichtungen 5, die natürlich von der Art des Systems abhängen, die jedoch Pumpen, Regelventile, Behandlungsvorrichtungen, wie z. B. Dialysegeräte oder Zentrifugen, Wärmvorrichtungen, Blasendetektoren und dergleichen enthalten können. Eine Abflußleitung 7 bildet den Rücklauf von den Behandlungsvorrichtungen 5, so daß ein Patient daran zur Behandlung angeschlossen werden kann. Die Gesamtvorrichtung wird durch entsprechende Steuerungen 9 gesteuert.

In dem in Fig. 1 dargestellten System ist es wichtig, daß an verschiedenen Punkten im Gesamtsystem der Druck überwacht wird. In der in Fig. 1 dargestellten Anordnung sind Drucküberwachungsvorrichtungen 11 und 13 zur Überwachung des Druckes in der Einlaufleitung 3 bzw. der Abflußleitung 7 vorgesehen. Jede der Drucküberwachungsvorrichtungen besteht aus einem Druckmeßblock 15 und 17, durch den die entsprechende Leitung 3 und 7 verläuft und der einen Druckwandler enthält, der elektrische Ausgangssignale an die zugehörigen Verstärker 19 und 21 liefert. Die Ausgangssignale der Verstärker 19 und 21 werden an die Steuerung 9 abgegeben und liefern die entsprechenden Steuer- und Alarmsignale, wenn die Drücke in den Leitungen 3 und 7 von den voreingestellten Grenzwerten abweichen.

Die eigentliche Drucküberwachungsvorrichtung ist im einzelnen in Fig. 2 gezeigt. Jeder der Druckmeßblocks 15, 17 besteht aus einem Block 23 mit einem Kanal 24, in die die entsprechende Flüssigkeitsleitung 25 (ein Rohr oder ein Schlauch) eingesetzt werden kann, wie dies aus der Schnittansicht in Fig. 2 zu erkennen ist. Da diese Leitungen oder Schläuche 3 und 7 normalerweise nur einmal benutzt werden, muß für jede Benutzung des gesamten Systems ein neuer Satz Leitungen oder Schläuche in die Druckmeßvorrichtungen eingesetzt werden. Es ist dabei ganz wesentlich, daß durch die erfindungsgemäß aufgebaute Meßvorrichtung festgestellt werden kann, daß der Schlauch oder die Leitung 25 richtig in den Block 23 eingesetzt ist. In einer der Seitenwände des Blocks 23 in Richtung auf den Kanal 24 ist ein druckempfindlicher Wandler 27 eingebaut. Die Einzelheiten dieses Wandlers sind nicht gezeigt, sie sind jedoch bekannt und sind im allgemeinen handelsübliche Wandler mit Widerstandsdehnungsmeßstreifen, die in Brückenschaltung angeordnet sind. Gemäß üblicher Praxis bei Brückenschaltungen aus Dehnungsmeßstreifen wird eine Stromquelle über Leitungen 29 und 31 über der Brückenschaltung angeschlossen. Die Ausgangssignale treten über den Ausgangsleitungen 33 und 35 auf und werden den Verstärkerschaltungen 19 zugeführt. Weitere Steuerstromkreise sind über Leitungen 37 und 39 an den Steuerungen 9 angeschlossen, und Ausgangssignale der Verstärkerschaltungen werden über Leitungen 41, 43 und 45 an die Steuerungen 9 abgegeben.

Ist die Leitung oder der Schlauch 25 richtig in den Block 23 eingesetzt, dann sind die Abmessungen und Anordnungen der Teile derart, daß die Leitung einen bestimmten Vorlastdruck von beispielsweise 0,21 kp/cm$^2$ bis 0,63 kp/cm$^2$ auf den Druckwandler 27 ausübt. Ist die Leitung jedoch nicht richtig eingesetzt, dann wird natürlich der richtige Vorlastdruck nicht erzielt.

Der Verstärker 19 ist in der Lage, das Vorlastsignal des Wandlers zu kompensieren und und damit eine Nullstellung festzulegen. Der Kompensationsbereich des Verstärkers wird dabei eng an dem gewünschten Vorlastbereich, beispielsweise von 0,21 kp/cm$^2$ bis 0,63 kp/cm$^2$ gehalten. Wenn daher die Vorlast des Druckwandlers wegen falsch eingesetzter Leitung 25 im Kanal 24 des Blockes 23 nicht innerhalb des erwarteten Vorlastbereiches liegt, dann kann der Verstärker das unter Vorlastbedingungen gelieferte Signal nicht zum Nullsignal machen.

In diesem Fall tritt auf der Ausgangsleitung 46 des Verstärkers 19 ein positives Signal auf und verhindert damit, daß das System gemäß Fig. 1 in seinen Betriebszyklus übergeht. Ein Warnsignal kann dann den Bediener der Vorrichtung darauf aufmerksam machen, daß die Leitung erneut eingelegt werden muß, damit der Anlaufzyklus wieder eingeleitet werden kann.

Wenn dann die Leitung 25 richtig in den Kanal 24 eingesetzt ist, und die Maschine oder das System in seinen Betriebszyklus übergeht, dann wird die Kompensationsschaltung des Verstärkers 19 durch ein über Leitung 39 dem Eingang des Verstärkers 19 zugeführtes Signal abgeschaltet. Der Verstärker 19 arbeitet nunmehr als Detektor für relative Druckänderungen und kann damit eine Druckabnahme von 0,1575 kp/cm$^2$ oder eine Druckzunahme von 0,3675 kp/cm$^2$ feststellen. Wenn eine dieser Druckänderungen eintritt, dann wird der Betriebszyklus der Maschine so lange beendet, bis die Ursache der Druckänderung festgestellt und beseitigt ist. Diese eine Druckabnahme oder Druckzunahme anzeigenden Signale werden bei Auftreten von dem Verstärker 19 über Ausgangsleitungen 41 und 43 abgegeben. Die verbleibende Signaleingangsleitung des Verstärkers 19 ist die Leitung 37, die die Taktsignale zuführt, die innerhalb der Steuerung 9 des in Fig. 1 gezeigten Systems erzeugt werden.

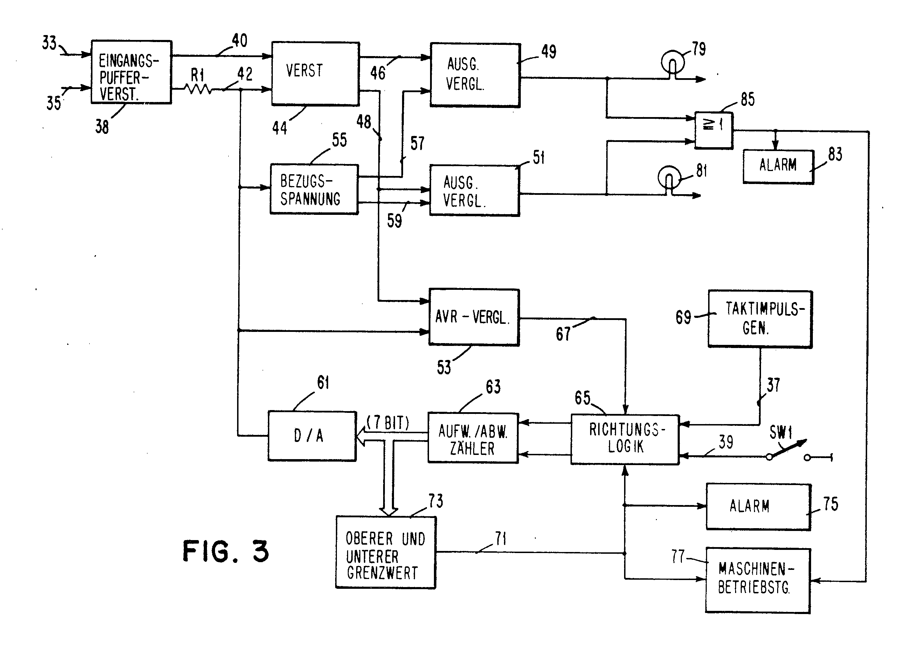

In Fig. 3 sind die in dem Verstärker 19 enthaltenen Schaltungen als Blockschaltbild dargestellt. Die vom Wandler kommenden Signalleitungen 33 und 35, die das vom Wandler 27 kommende Signal führen, sind am Eingang eines Eingangspufferverstärkers 38 angeschlossen. Der genaue Aufbau dieses Pufferverstärkers ist nicht dargestellt, da er kein Teil der Erfindung ist und dafür bekannte Schaltungen verwendet werden können. Dies trifft auch für die übrigen Teile der Schaltung gemäß Fig. 3 zu, wobei die einzelnen Blöcke Schaltungen bezeichnen, die für sich allein bekannt sind und beim Entwerfen der Schaltung entsprechend ausgewählt werden können.

Am Ausgang des Eingangspufferverstärkers 38 sind eine Signalleitung 40 und eine mit einem Widerstand R1 in Reihe geschaltete Bezugssignalleitung 42 angeschlossen. Die Pufferverstärker sind vorgesehen, damit der Rest der Schaltung für Impedanztoleranzen des Druckwandlers nicht empfindlich ist.

Leitungen 40 und 42 sind an den Eingängen eines Verstärkers 4 angeschlossen, dessen Ausgangssignale über Leitungen 46 und 48 abgegeben werden. Leitung 46 ist am Eingang einer Ausgangsvergleichsschaltung 49 angeschlossen, und Leitung 48 ist am Eingang einer Ausgangsvergleichsschaltung 51 angeschlossen sowie an einem Eingang einer Vergleichsschaltung 53 für die automatische Verstärkungsregelung. Die Bezugsspannungsleitung 42 ist weiterhin am Eingang eines Bezugsspannungsgenerators 55 angeschlossen, der ausgangsseitig über Leitungen 57 bzw. 59 an den Vergleichsschaltungen 49 und 51 angeschlossen ist. Die Bezugsspannungsleitung 42 ist außerdem als eine der beiden Eingangsleitungen an der Vergleichsschaltung 53 für die automatische Verstärkungsregelung angeschlossen. Das auf der Bezugsspannungsleitung 42 liegende Signal ist ein Analogsignal, das als Ausgangssignal von einem Digital-Analogwandler 61 kommt, dem wiederum ein 7-Bit-Digitalsignal zugeführt wird, das als Ausgangssignal von einem digitalen Aufwärts-/Abwärtszähler 63 geliefert wird.

Der Aufwärts-/Abwärtszähler 63 wird durch Impulse angesteuert, die durch eine Gruppe kombinatorischer Schaltungen geliefert werden, welche als Richtungslogik 65 bezeichnet werden. Die Richtungslogik 65 liefert Impulse, die bewirken, daß der Zähler 63 entweder vorwärts oder rückwärts zählt, je nachdem ob am Eingang der Richtungslogik Signale ankommen oder nicht. An diesen Eingängen liegt eine Signalleitung 67, die das Ausgangssignal der Vergleichsstufe 53 für automatische Verstärkungsregelung führt, eine Taktsignalleitung 37, die die durch einen Taktimpulsgenerator 69, der in der Steuerung 9 liegt, gelieferten Taktimpulse führt, und eine durch einen Schalter SW1 in der Steuerung 9 betätigte Steuersignalleitung, wobei dieser Schalter dann geschlossen ist, wenn die Maschine in ihren Betriebszyklus übergeht, sowie eine innere Verbindungsleitung 71, die das Ausgangssignal der den unteren bzw. oberen Grenzwert liefernden Schaltung 73 führt. Die Leitung 71 ist außerdem an einer Alarmvorrichtung 75 und an der Maschinenbetriebssteuerung 77 angeschlossen, so daß ein auf der Leitung 71 auftretendes Signal nicht nur die Alarmvorrichtung 75 betätigt, sondern außerdem den Betriebszyklus der Maschine abschaltet. Die unteren und oberen Grenzwerte 73 werden durch eine kombinatorische Dekodierlogik dargestellt, die dann ein Ausgangssignal auf Leitung 71 liefert, wenn am Ausgang des Aufwärts-/Abwärtszählers 63 ein Zählwert auftritt, der einen vorbestimmten Zählwert überschreitet.

Die Ausgangssignale der Vergleichsschaltungen 49 und 51 werden Anzeigelampen 79 und 81 und einer gemeinsamen Alarmvorrichtung 83 zugeführt. Diese Anzeigelampen mit zugehöriger Alarmvorrichtung zeigen dem Bediener während eines Betriebszyklus an, ob der Druck in der Leitung um 0,1575 kp/cm² abgesunken oder auf über 0,3675 kp/cm² angestiegen ist.

Die von den Vergleichsschaltungen 49 und 51 kommenden Signale werden der Alarmvorrichtung 83 über ein ODER-Glied 85 zugeführt, das außerdem ein Ausgangssignal an die Maschinenbetriebssteuerung 77 abgibt und damit den Betriebszyklus der Maschine anhält, wenn der Druck die angegebenen Grenzen nach unten oder oben überschreitet.

Im Betrieb wird also der Maschinenbediener den entsprechenden Leitungsabschnitt vor Anlauf des gesamten Systems in den Druckmeßblock einsetzen. Wenn das System den Anlaufzyklus durchläuft, dann überprüft die Druckmeßvorrichtung den Druck in den Flüssigkeitsleitungen, und wenn das Ausgangssignal anzeigt, daß der Druck innerhalb der vorgegebenen Grenzen von 0,21 kp/cm² und 0,63 kp/cm² liegt, dann kann die Maschine in den Betriebszyklus übergehen. Wenn während des Betriebszyklus die vorgegebenen Druckgrenzen in der einen oder der anderen Richtung überschritten werden, dann leuchtet entweder die Warnlampe 79 oder die Warnlampe 81 auf, und die Alarmvorrichtung 83 wird ein Warnsignal abgeben, während außerdem der Maschinenbetriebssteuerung 77 ein Signal zugeleitet wird, das die Arbeitsweise des außerhalb des Körpers befindlichen Kreislaufsystems anhält.

Wenn sich die Maschine in ihrem Betriebszyklus befindet, dann kann der Verstärker wegen der Betätigung des Schalters SW1, der die Richtungslogik 65 abschaltet, seine Kompensationswirkung nicht ausüben. Der Verstärker arbeitet dann als Detektor zur Feststellung einer relativen Druckänderung bei einer Abnahme von 0,1575 kp/cm² oder bei einer Druckzunahme von 0,3675 kp/cm². Wenn eine der beiden Druckänderungen eintritt, wird der Betriebszyklus der Maschine beendet. Es ergibt sich somit klar, daß durch die Erfindung ein Drucküberwachungssystem geschaffen worden ist, das so aufgebaut ist, daß zunächst sichergestellt ist, daß das System, richtig arbeitet, bevor die damit verbundene Apparatur voll in Betrieb genommen werden kann.

**Patentansprüche**

1. Vorrichtung für die Überwachung des Druckes eines strömenden flüssigen Mediums in einem biegsamen Rohr oder Schlauch (25) zur Feststellung einer Abweichung des jeweiligen Istwertes des Druckes von durch einen oberen und einen unteren Bezugssignalwert begrenzten Sollwerten, mit einem an das Rohr oder den Schlauch angekoppelten druckempfindlichen Wandler (27), Vergleichsschaltungen (49, 51) zum Vergleich des Ausgangssignals des Wandlers mit dem oberen und unteren Bezugssignalwert, sowie einer Steuerschaltung (37, 39, 53 – 77) zur Einstellung der Bezugssignalwerte in Abhängigkeit vom Istwert des Druckes während einer ersten Betriebsphase und zur Bereithaltung der eingestellten Bezugssignalwerte während einer zweiten Betriebsphase, dadurch gekennzeichnet, daß ein Stützblock (23) mit einem für die Aufnahme des Rohrs oder Schlauchs (25) bestimmten Kanal (24) vorgesehen ist, in dessen Seitenwand der Wandler (27) in der Weise angeordnet ist, daß das in den Stützblock eingelegte Rohr bzw. der Schlauch einen vorbestimmten Vorlastdruck auf den Wandler ausübt,

daß die Steuerschaltung eine weitere Vergleichsschaltung (53) aufweist, die ein in Abhängigkeit von ihrem Ausgangssignal erzeugtes, der Einstellung der Bezugssignalwerte dienendes Steuersignal mit dem Ausgangssignal des Wandlers (27) vergleicht,

daß eine logische Schaltung (73, 75) zur Feststellung und Anzeige der Unter- bzw. Überschreitung eines vorgegebenen unteren bzw. oberen Grenzwertes durch das Steuersignal während der ersten Betriebsphase vorgesehen ist, wobei der obere und untere Grenzwert in Abhängigkeit von den Bereichsgrenzen des zulässigen Vorlastdrucks gewählt sind.

2. Vorrichtung nach Anspruch 1, dadurch gekennzeichnet, daß zwischen dem Wandler (27) und der weiteren Vergleichsschaltung (53) ein Verstärker (44) eingeschaltet ist, wobei das Steuersignal an einem Eingang des Verstärkers (44) angeschlossen ist und damit dessen Ausgangssignal mit beeinflußt.

3. Vorrichtung nach Anspruch 2, dadurch gekennzeichnet, daß die Steuerschaltung mit einem Taktimpulsgenerator (69), einem Aufwärts-/Abwärtszähler (63) und digitalen logischen Schaltung (65) versehen ist, die an dem Taktimpulsgenerator (69) angeschlossen sind und in Abhängigkeit vom Ausgangssignal der weiteren Vergleichsschaltung (53) Taktimpulse an den Zähler (63) liefern, und daß der Zähler (63) ausgangsseitig mit einem Digital-Analogwandler (61) verbunden ist, an dessen Ausgang das Steuersignal in analoger Form ansteht.

4. Vorrichtung nach Anspruch 3, dadurch gekennzeichnet, daß Sperrschaltmittel vorgesehen sind, durch die zur Beendigung der ersten Betriebsphase die digitalen logischen Schaltungen (65) abschaltbar sind, wodurch die Zufuhr weiterer Taktimpulse vom Taktimpulsgenerator (69) an den Zähler (63) verhindert wird.

5. Vorichtung nach den Ansprüchen 3 und 4, dadurch gekennzeichnet, daß die logische Schaltung (73, 75) einen Decodierer für die vom Zähler kommenden, den oberen bzw. unteren Grenzwert überschreitenden Ausgangssignale aufweist.

6. Vorrichtung nach Anspruch 5, dadurch gekennzeichnet, daß zur Betätigung der Sperrschaltmittel ein Schalter (SW1) vorgesehen ist.

### Claims

1. Device for monitoring the pressure of a flowing liquid medium in a flexible pipe or tube (25) for detecting a deviation of the respective actual value of the pressure from nominal values limited by an upper and a lower reference signal value, with a pressure-sensitive transducer (27) coupled to the pipe or tube, comparing circuit means (49, 51) for comparing the output signal of the transducer with the upper and lower reference signal value, and with a control circuit (37, 39, 53–77) for setting the reference signal values as a function of the nominal pressure value during a first operating phase and for providing the set reference signal values during a second operating phase, characterized in that a supporting block (23) with a channel (34) for receiving the pipe or tube (25) is provided, the transducer (2) being arranged in its wall in such a manner that the pipe or tube inserted in the supporting block exerts a predetermined initial load pressure onto the transducer, that the control circuit comprises another comparing circuit (53) for comparing a control signal generated as a function of its output signal and serving for setting the reference signal value with the output signal of the transducer (27), that a logic circuit (73, 75) determining and displaying the exceeding by the control signal of a given lower or upper limiting value during the first operating phase ist provided, the upper and lower limiting value being determined as a function of the limits of the range of admissible initial load pressures.

2. Device as claimed in claim 1, characterized in that between the transducer (27) and the other comparing circuit (53) an amplifier (44) is provided, whereas the control signal is connected to an input of the amplifier (44) and thus also controls the output signal of the latter.

3. Device as claimed in claim 2, characterized in that the control circuit comprises a clock pulse generator (69), an up-down counter (63), and a digital logic circuit (65), which latter ist connected to the clock pulse generator (69) and supply clock pulses to the counter (63) as a function of the output signal of the other comparing circuit (53), and that the counter (63) is connected with its output to a digital-to-analog converter (61), its output being the control signal in analog form.

4. Device as claimed in claim 3, characterized in that inhibiting means are provided by means of which at the end of the first operating phase the digital logic circuits (65) can be switched off, thus preventing further clock pulses from the clock pulse generator (69) reaching the counter (63).

5. Device as claimed in claims 3 and 4, characterized in that the logic circuit (73, 75) comprises a decoder for the output signals from the counter which exceed the upper or the lower limiting values.

6. Device as claimed in claim 5, characterized in that for operating the inhibiting means a switch (SW1) is provided.

### Revendications

1. Dispositif pour le contrôle de la pression d'un milieu liquide en mouvement dans un tuyau souple (25) pour détecter une déviation de la valeur réelle de la pression par rapport à des valeurs nominals limitées par des valeurs de signal de référence supérieure et inférieure, comportant un transducteur (27) sensible à la pression et connecté au tuyau, des circuits de comparaison (49, 51) pour comparer le signal de sortie du transducteur avec les valeurs de signal de référence supérieure et inférieure, ainsi qu'un circuit de commande (37, 39, 53 − 77) pour ajuster les valeurs de signal de référence en fonction de la valeur réelle de la pression durant une première phase de fonctionnement et pour rendre disponibles les valeurs de signal de référence ajustées durant une seconde phase de fonctionnement, dispositif caractérisé en ce que il est prévu un bloc de support (23) avec un canal (24) destiné à recevoir le tuyau (25), un transducteur (27) étant disposé dans la paroi latérale de ce canal de telle sorte que le tuyau inséré dans le bloc de support exerce une pression de charge initiale prédéterminée sur le transducteur, en ce que le circuit de commande comporte un autre circuit de comparaison (53) qui compare un signal de commande généré en fonction de son signal de sortie et servant à ajuster les valeurs de signal de référence avec le signal de sortie du transducteur (27), et en ce qu'il est prévu un circuit logique (73, 75) pour déterminer et indiquer le dépassement d'une valeur limite supérieure ou inférieure par le signal de commande durant la première phase de fonctionnement, les valeurs limites supérieure et inférieure étant choisies en fonction des limites de pression de charge initiale admissible.

2. Dispositif selon la revendication 1, caractérisé en ce qu'un amplificateur (44) est connecté entre le transducteur (27) et le circuit de comparaison supplémentaire (53), le signal de commande étant appliqué àune sortie de l'amplificateur (44) de façon à influencer le signal de sortie de ce dernier.

3. Dispositif selon la revendication 2, caractérisé en ce que le circuit de commande comporte un générateur d'impulsions de chronologie (69), un compteur d'incrémentation/décrémentation (63) et un circuit logique numérique (65), ce dernier étant connecté au générateur d'impulsions de chronologie (69) et fournissant en fonction du signal de sortie du circuit de comparaison supplémenttaire (53) des impulsions de chronologie au compteur (63), et en ce que la sortie du compteur (63) est connectée à un convertisseur numérique-analogique (61) à la sortie duquel est appliqué le signal de commande sous forme analogique.

4. Dispositif selon la revendication 3 caractérisé en ce qu'il comporte des moyens d'inhibition qui permettent de déconnecter les circuits logiques numériques (65) à la fin de la première phase de fonctionnement, ce qui empêche que d'autres impulsions de chronologie en provenance du générateur d'impulsions de chronologie (69) ne soient appliquées au compteur (63).

5. Dispositif selon la revendications 3 et 4, caractérisé en ce que le circuit logique (73, 75) comporte un décodeur pour les signaux de sortie venant du compteur et dépassant la valeur limite supérieure ou inférieure.

6. Dispositif selon la revendication 5, caractérisé en ce qu'un commutateur (SW1) est prévu pour activer les moyens d'inhibition.

**FIG. 1**

**FIG. 2**

FIG. 3